# EUROPEAN PATENT SPECIFICATION

(11) **EP 1 781 199 B1**
(45) Date of publication and mention of the grant of the patent: **30.08.2017**
(21) Application number: 05758879.0
(22) Date of filing: 13.06.2005
(51) Int. Cl.: A61B 18/18

(54) **ABLATION CATHETERS**
ABLATIONSKATHETER
CATHETERS D'ABLATION

(30) Priority: 14.06.2004 US 867519
(43) Date of publication of application: 09.05.2007
(73) Proprietor: THE REGENTS OF THE UNIVERSITY OF MICHIGAN, Ann Arbor, Michigan 48109-1280 (US)
(72) Inventor: ORAL, Hakan, Ann Arbor, MI 48108 (US); MORADY, Fred, Ann Arbor, MI 48105 (US)
(74) Representative: Glawe, Delfs, Moll
(86) International application number: PCT/US2005/020845
(87) International publication number: WO 2005/122939

(56) References cited:
- US-A- 5 156 151
- US-A- 5 156 151
- US-A- 5 680 860
- US-A- 5 680 860
- US-A- 5 687 723
- US-A- 5 687 723
- US-A1- 2004 082 947
- US-B2- 6 572 612
- US-B2- 6 572 612

## Description

### FIELD OF THE INVENTION

The present description relates generally to catheters and methods for performing targeted tissue ablation in a subject. In particular, the present description provides devices comprising catheters having distal ends configured to treat two dimensional regions of target tissue, including deployable distal ends, and methods for treating conditions (e.g., cardiac arrhythmias) with these and similar devices. The invention is set out in the appended claims.

### BACKGROUND OF THE INVENTION

Mammalian organ function typically occurs through the transmission of electrical impulses from one tissue to another. A disturbance of such electrical transmission may lead to organ malfunction. One particular area where electrical impulse transmission is critical for proper organ function is in the heart. Normal sinus rhythm of the heart begins with the sinus node generating an electrical impulse that is propagated uniformly across the right and left atria to the atrioventricular node. Atrial contraction leads to the pumping of blood into the ventricles in a manner synchronous with the pulse.

Atrial fibrillation refers to a type of cardiac arrhythmia where there is disorganized electrical conduction in the atria causing rapid uncoordinated contractions which result in ineffective pumping of blood into the ventricle and a lack of synchrony. During atrial fibrillation, the atrioventricular node receives electrical impulses from numerous locations throughout the atria instead of only from the sinus node. This overwhelms the atrioventricular node into producing an irregular and rapid heartbeat. As a result, blood pools in the atria that increases a risk for blood clot formation. The major risk factors for atrial fibrillation include age, coronary artery disease, rheumatic heart disease, hypertension, diabetes, and thyrotoxicosis. Atrial fibrillation affects 7% of the population over age 65.

Atrial fibrillation treatment options are limited. Lifestyle change only assists individuals with lifestyle related atrial fibrillation. Medication therapy assists only in the management of atrial fibrillation symptoms, may present side effects more dangerous than atrial fibrillation, and fail to cure atrial fibrillation. Electrical cardioversion attempts to restore sinus rhythm but has a high recurrence rate. In addition, if there is a blood clot in the atria, cardioversion may cause the clot to leave the heart and travel to the brain or to some other part of the body, which may lead to stroke. What are needed are new methods for treating atrial fibrillation and other conditions involving disorganized electrical conduction.

Description of the background art. Nademanee et al. (2004), Journal of the American College of Cardiology,43;11: 2044-2053 describes the use of conventional mapping and ablation protocols for treating atrial fibrillation.

The following U.S. Patents describe cardiac ablation and other intracardiac electrode structures: 4,112,952; 5,083,565; 5,156,151; 5,255,679; 5,462,545; 5,536,267; 5,575,766; 5,575,810; 5,582,609; 5,626,136; 6,001,093; 6,064,902; 6,071,274; 6,106,522; 6,129,724; 6,146,379; 6,171,306; 6,226,542; 6,241,754; 6,371,955; 6,447,506; 6,454,758; 6,460,545; 6,471,699; 6,500,167; 6,514,246; 6,540,744; 6,551,271; 6,574,492; 6,607,520; 6,628,976; and 6,640,120 and published applications US 2003/0195407 A1 and US2003/0199746A1. Two abstracts relating to the present invention naming the inventors as authors were published on November 5, 2002 (see Oral H, et al., Circulation 106(19): II-516) and October 28, 2003 (see Oral H, et al., Circulation 108(17):IV-618;). US patent US2004/082947 discloses an ablation catheter having a deployable spiral or umbrella tip.

### BRIEF SUMMARY OF THE INVENTION

The present description provides methods and apparatus for performing cardiac ablation procedures for treating cardiac arrhythmias, particularly atrial fibrillation. Atrial fibrillation is believed to often result from the presence of regions of disorganized electrical conduction which may be found distributed over numerous locations on the wall of an atrial or other heart chamber. The present description provides for localization and/or selective ablation of such regions on the atrial wall and/or other endocardial surfaces in order to treat atrial fibrillation and other arrhythmias. The mapping and ablation systems of the present description identify areas of the heart that are critical to the initiation and maintenance of arrhythmias, referred to hereinafter as "critical sites". Critical sites are characterized by one or more of the following:
- 1.: high frequency electrograms
- 2.: electrograms with a short cycle length
- 3.: rapid repetitive electrical activity
- 4.: aberrant pulse generation
- 5.: fractionated electrograms
- 6.: areas of slow conduction
- 7.: areas of anchor points
- 8.: areas of pivot points
- 9.: areas of turning points for electrical circuits
- 10.: sites of nervous innervation of the heart
- 11.: sites of vagal or parasympathetic nerve terminals, ganglia or neurons
- 12.: sites of sympathetic nerve terminals, ganglia or neurons
- 13.: reentrant circuits
- 14.: aberrant pathways

While the present description is particularly useful for treating atrial fibrillation which originates in the artia, it is also useful for tracking other arrhythmias originating in other heart chambers, including supra ventricular tachycardias; inappropriate sinus tachycardia; atrial tachycardia; atrial flutter (right and left atrial, typical and atypical); accessory pathways; WPW; ventricular tachycardia; and ventricular fibrillation.

The present description also provides for mapping of the heart chamber (including but not limited to atria) and may be performed with the novel catheters of the present description that have an array system for placement of electrodes on the endocardial or in some instances an epicardial surface of the heart. In some embodiments, access to the endocardium is via intravascular access protocols which are well known. Access to the epicardium may be achieved either percutaneously through a subxiphoid approach where a sheath is advanced over a guide-wire introduced into the pericardial space through a fine needle or surgically.

The present description provides both methods and devices for performing cardiac ablation over a two-dimensional endocardial surface, including but not limited to, surfaces in an atrial chamber of the heart. Such cardiac ablation forms a pattern of multiple lesions selected to disrupt the aberrant conductive pathways responsible for atrial fibrillation and other cardiac arrhythmias. The methods and devices typically utilize an electrode array comprising a plurality of individual electrode elements, typically from 2 to 50 (e.g., 6 to 30) individual elements, often from 8 to 20 individual elements, and more often from 10 to 20 individual elements. The electrode elements are preferably distributed over an area in the range from 1 cm² to 12 cm², usually from 2 cm² to 12 cm², and often from 4 cm² to 12 cm². The electrode elements are preferably distributed in a generally uniform manner over the area of the electrode array, and the array is arranged to conform to regular and irregular surfaces of the types that are encountered in a heart chamber, particularly in the atrium. The electrode elements may be arranged generally symmetrical or in another regular pattern about a center point and/or a line of symmetry, but such symmetrical or regular distribution is not essential.

In preferred aspects of the present description, the catheters is useful for both mapping and ablation. In particular, for use in mapping, the individual electrode elements are configurable in a unipolar or bipolar pattern to allow mapping of the tissue against which the electrode array is engaged. To allow ablation, the electrode elements are usually configurable in a unipolar pattern, although in some circumstances a bipolar arrangement is preferred. By "bipolar," it is meant that the individual electrodes may be configured so that at least one of the electrodes is connected to external detection (mapping) or ablation circuitry as the common lead, while at least some of the remaining electrode elements are connected as the active or sensing leads to the detection and/or ablation circuitry. The detection circuitry can then be used to map the region contacted by the electrode array to identify aberrant circuit paths therein, and the ablation circuitry can be used to ablate some or all of the detected aberrant paths. By "unipolar," it is meant that the electrode elements is connected to an external detection or high frequency, usually radiofrequency source which is also connectable to an external or dispersive electrode element which is placed on the patient's back, thighs, or elsewhere. The individual electrode elements can then be sensed and/or powered individually or in groups with the dispersion of electrode acting as the counterelectrode.

Thus, the catheters of the present description can be used to first map a region of the atrial wall or other endocardial tissue to locate aberrant conductive pathways. The same catheter can then be used to treat the aberrant conductive pathways by tissue ablation at locations selected based on the previous mapping procedure. The ablation can be performed either with or without repositioning of the catheter. That is, the aberrant conductive pathways may be treated by energizing some or all of the electrode elements of the array in their original positions where they were during the mapping procedure. Alternatively, based on the location of the aberrant pathways, the catheter may be moved to one or more subsequent positions surrounding the aberrant pathway and thereafter energize to ablate the tissue to create the desired lesion pattern.

In one embodiment of the present description, a cardiac ablation catheter comprises a catheter body and a two-dimensional electrode array deployable from a distal region, typically through a distal port or a side port of the catheter body. The distal end of the catheter body is preferably steerable to permit selective placement within a cardiac chamber. The two-dimensional electrode array preferably comprises a plurality of electrode elements distributed over an area in the range from 1 cm² to 12 cm², preferably from 2 cm² to 12 cm², more preferably from 4 cm² to 12 cm². The electrode elements are engageable against an endocardial surface in the heart chamber to map and/or ablate critical sites on said surface. Preferably, the electrode elements are mounted on a flexible or resilient surface so that the arrangement in the elements can conform to a target endocardial surface to promote electrical contact between the electrode elements and the surface. The surface should be sufficiently flexible so that contact between the electrode elements and the endocardial surface can be achieved with a minimum force to reduce the risk of unintentional damage to the endocardial surface.

In a preferred embodiment of the present description, the electrode elements is disposed on a support which is shiftable between at a storage configuration and a deployed configuration. In the deployed configuration, the support structure positions the electrode elements generally over a "plane" which is generally perpendicular to the axis of the distal end of the catheter body. By "plane," it is meant that the electrode element arrangement is disposed generally laterally with respect to the catheter body. The arrangement need not be completely flat and may in fact be slight concave or irregular so that the outer peripheral edge contacts the tissue surface first. The support is sufficiently conformable to the surface so that the individual electrode elements may contact the surface by advancing the distal end of the catheter body against or toward the surface in order to press the electrodes there against. In a first example, the support surface may comprise at least one elongate member that is extendible from a radially constrained storage configuration within the catheter body to a radially expanded deployed configuration outside the catheter body. The elongate member may be formed from an elastic material, such as a shape memory alloy, spring stainless steel, or the like, and may be in the form of wire, ribbon, cable, strut, or the like. In some embodiments, the elongate member assumes a spiral or helical configuration when outside of the catheter body. In other embodiments the support comprises a plurality of elongate members arranged to open radially from a common point outside the catheter body, referred to hereinafter as an "umbrella" configuration. Other configurations, such as zig-zag patterns, serpentine patterns, star burst patterns, and the like, also find use.

In preferred embodiments, an electrode array typically includes from 6 to 30 electrode elements, usually from 8 to 20 electrode elements, and often from 10 to 20 electrode elements. The electrode elements typically are electrically.isolated from each other so that they be separately connectable to external detection and/or energizing circuitry. In this way, the individual electrodes can be operated in a bipolar or unipolar fashion depending on how the catheter is to be used. Of course, in other configurations, certain pairs or groups of electrodes are commonly connected, but the electrode array preferably includes at least one electrode which is isolated from the remaining electrodes in order to permit bipolar operation, particularly for mapping.

The individual electrode elements may have a wide variety of configurations. For example, the electrode elements may comprise helically coiled structures formed over an elongate member which forms the support structure. Alternatively, the electrode elements could comprise plates, bands, tubes, spheres, or other conventional electrode structures of the type used in medical devices for tissue ablation. In preferred embodiments, the electrode elements typically have a maximum length or other dimension in the range from 2 mm to 20 mm, typically from 4 mm to 8 mm and preferably from 3 mm to 5 mm, and are spaced-apart by a distance in the range from 1 mm to 10 mm, typically from 1 mm to 6 mm and preferably from 1 mm to 5 mm. Most commonly, the electrode elements are elongated, where the maximum dimension is length. In the specific case of helically wound electrical elements, the length from end to end of the helix may be in the ranges set forth above, while the diameter of the helix is typically from 5 mm to 40 mm, usually from 10 mm to 30 mm.

In a further aspect of the present description, a method for ablating an endocardial surface to treat an arrhythmia or for other purposes, comprises engaging a two-dimensional electrode array against the surface and delivering electrical energy through at least some of the electrode elements to the surface. Electrode arrays engaged against an endocardial surface preferably have an area in the range from 1 cm² to 12 cm², typically from 2 cm² to 12 cm², more typically from 4 cm², to 12 cm². An endocardial surface is typically an atrial surface which is treated for atrial fibrillation. The atrial service which is treated may be remote from the pulmonary vein os. the electrical energy is delivered through individual ones of the electrode elements which are selected to ablate tissue proximate an aberrant conductive pathway in the tissue, such as a rotor or an equivalent aberrant structure in the atrial wall. Electrical energy may be delivered through any or all of the electrode elements, where energy delivered through multiple electrode elements may be delivered simultaneously, sequentially, or in some combination thereof.

Optionally, the methods further (or alternatively) comprise locating critical site(s) on the atrial or other endocardial surface prior to delivering the ablative electrical energy. Locating typically comprises engaging the two-dimensional electrode array against an atrial or other endocardial wall surface and detecting electrical signals through the array. Radiofrequency or other ablative electrical energy may then be delivered simultaneous or sequentially through at least some of the electrode elements in the electrode array, where the particular electrode elements may be selected based on proximity to the detected location of the critical site(s). The electrical energy may be delivered through the electrode array, either with or without repositioning of the array. For example, after the array has been used for detecting and locating the site of a critical site, some or all of the electrode elements can be used to deliver ablative electrical energy to create a desired lesion pattern. Optionally, the catheter may then be repositioned to one or more locations about the critical site and additional ablative conductive energy delivered through any or all of the electrode elements at any or all of the further contact sites.

Thus, in a particularly preferred aspect of the present description, atrial fibrillation may be treated by first locating a critical site on an atrial wall and thereafter creating a pattern of lesions over a two-dimensional region of the atrial wall selected to disrupt the aberrant conductive pathway. Locating typically comprises engaging a two-dimensional electrode array against the atrial wall surface and detecting electrical signals from the array. Creating a pattern of lesions typically comprises delivering electrical or other energy through at least some of a plurality of electrode elements within the electrode array which was used for mapping and locating the critical site. Other aspects of the preferred method have been described previously.

Mapping according to the present description, identifies critical sites as identified above that are responsible for initiation or perpetuation/maintenance of an arrhythmia including but not limited to atrial fibrillation. One approach includes but is not limited to analysis of electrograms recorded from electrodes positioned on the array system of the catheters to identify such sites that have a high frequency and/or rapid repetitive electrical activity, and/or short cycle lengths, and/or fractionated and/or continuous electrograms. Another approach is to identify sites of autonomic innervation of the heart and to ablate these sites. An imbalance between the sympathetic and parasympathetic innervation of the heart, and/or over stimulation of either or both, particularly the parasympathetic component of the autonomic inputs, may cause arrhythmias in particular atrial fibrillation. Identification and ablation of these neural inputs, particularly parasympathetic or vagal inputs to the heart chamber may eliminate the arrhythmia, atrial fibrillation in particular. Such neural inputs have ganglia over the epicardial surface of the heart from which nerve terminals originate and spread over the heart. Such sites may be identified by delivering electrical stimuli from the endocardial or epicardial surface of the heart. The frequency and output of these stimuli can be programmed such that the stimuli do not result in excitation of the myocardial tissue. However they may be sufficient to stimulate the nerve terminals or ganglia and evoke a parasympathetic or sympathetic response. When there is stimulation of the afferent nerve terminals particularly the vagal terminals or ganglia, there often is slowing of the heart rate or complete asystole and a decrease in blood pressure. Therefore such sites can easily be identified by delivering stimuli via the electrodes of the catheters described herein. These stimuli are generated by an external muscle/nerve stimulator which is commercially available. Such a stimulator may be connected to the catheter system through a switch box which enables toggling of the input to and output from the electrodes, among the electrophysiologic recording system, energy source for ablation, and high frequency generator (nerve stimulator). Once these sites of neural input or autonomic innervation are identified they can be ablated through the same electrodes without necessarily changing the position of the catheter. Because anatomical location of the neural inputs and ganglia may have variability among patients and because these sites may be spread over an area of several square centimeters, the catheters described herein provide a unique design to identify and ablate such neural inputs, or sites of autonomic innervation or ganglia, particularly parasympathetic or vagal, over a wide area covered by the array system of the catheters.

In some embodiments, the present description, provides a device (*e.g*., for performing at least one function at an internal site in a subject), comprising an elongate catheter body. The elongate catheter body may comprise a proximal end, a distal end (typically steerable), and a spiral tip, wherein the spiral tip is configured for tissue ablation. In addition, the spiral tip may be mounted at the distal end of the elongate catheter body. The spiral tip is capable of expansion and contraction. In further embodiments, the spiral tip is mounted either centrally or peripherally with the elongate catheter body. In preferred spiral top embodiments, the spiral tip is configured to create spiral lesions in targeted body tissue.

In other embodiments, the device comprises individual electrode elements comprising conductive coils on the spiral tip. In particular embodiments, the conductive coils comprise at least one conductive coil measuring 2 to 20 millimeters in size. Alternatively, in some embodiments the device comprises conductive plates on the spiral tip. In particular embodiments, at least one such conductive plate may measure 2 to 20 millimeters in size.

Embodiments with a spiral tip electrode array may have the spiral tip positioned generally perpendicularly to the distal end of the elongate catheter body. Such perpendicular orientation of the spiral electrode array facilitates engagement of the array against target tissue so that the spiral generally conforms to the tissue and provides good electrical contact with the individual electrode elements on the spiral support element. In addition, in some embodiments, the spiral tip may comprise a plurality of loops. In other embodiments, the spiral tip loops may be separated by gaps. In particular embodiments, such gaps may measure less than 10 millimeters.

Some embodiments may also comprise a handle attached to the proximal end of the elongate catheter body. In further embodiments, the handle is configured to control expansion or contraction of the spiral tip as well as flexion and extension of the catheter tip. In yet other embodiments, the device further comprises an energy source configured to permit emission of energy from the spiral tip.

In some embodiments, the present description, provides an elongate catheter body, wherein the elongate catheter body comprises a proximal and distal ends, a lumen therebetween and an umbrella tip body. In some embodiments, the umbrella tip body comprises a central post, optionally carrying an electrode element, and a plurality outer arms. In preferred embodiments, the umbrella tip body is configured for tissue mapping and ablation. In other embodiments, the umbrella tip body is mounted at the distal end of the elongate catheter body.

In some embodiments, the present description, provides a central post extending from a distal region of said elongate catheter body, typically through a distal port or a side port. In other embodiments, the plurality of outer arms attaches at distal and proximal ends of the central post. In still other embodiments, the electrode array is deployed from a malecot braid, or other structure which radially expands when axially shortened.

In other embodiments, the device comprises individual electrode elements comprising conductive coils on the outer arms. In particular embodiments, the conductive coils comprise at least one conductive coil measuring 2 to 20 millimeters in size. Alternatively, in some embodiments the device comprises conductive plates on the outer arms. In particular embodiments, at least one such conductive plate measures 2 to 20 millimeters in size. In preferred embodiments, the umbrella tip is configured to create radial lesions in body tissue.

Some embodiments also comprise a handle attached to the proximal end of the elongate catheter body. In further embodiments, the handle is configured to control expansion or contraction of the umbrella tip body as well as flexion and extension of the catheter tip. In yet other embodiments, the device further comprises an energy source configured to permit emission of energy from the umbrella tip body.

In some embodiments, the present description provides a method of treating body tissues. In such embodiments, the method comprises the steps of providing a device, and detailed treatment steps. In other embodiments, the present description provides an energy source, such as a radiofrequency source, a microwave source, or in some instances a cryogenic or chemical ablation source.

In particular embodiments, the device comprises an elongate catheter body, wherein the elongate catheter body comprises a proximal end and a distal end, and also a spiral tip, wherein the spiral tip is configured for tissue ablation, the spiral tip mounted at the distal end of the elongate catheter body, and is capable of expansion and contraction.

In other particular embodiments, the device comprises an elongate catheter body, wherein the elongate catheter body comprises a proximal end and a distal end, and also an umbrella tip body, wherein the umbrella tip body is configured for tissue ablation, the umbrella tip body is mounted at the distal end of the elongate catheter body, and the umbrella tip body is capable of expansion and contraction. In still further embodiments, the umbrella tip comprises a central post, and a plurality of outer arms.

In some embodiments, the detailed treatment steps comprise the inserting of the catheter through a major vein or artery, the guiding of the catheter to the selected body tissue site by appropriate manipulation through the vein or artery, the guiding of the catheter to the selected body tissue site, the positioning of the device with the selected body tissue; and the releasing of energy from the device into the body tissue.

In particular embodiments, the detailed treatment steps are specific for treating atrial fibrillation, and comprise the inserting of the catheter through a major vein or artery, the guiding of the catheter into the atria of the heart by appropriate manipulation through the vein or artery, the guiding of the catheter to the target atrial region, the positioning the device with the targeted atrial region; and a releasing of energy from the device into the targeted atrial region.

In still further embodiments, the detailed treatment steps are specific for treating cardiac arrhythmias, and comprise the inserting of the catheter through a major vein or artery, the guiding of the catheter into the heart by appropriate manipulation through the vein or artery, the guiding of the catheter to the targeted heart region, typically the atrium or other heart chamber, the positioning of the device with the targeted heart region; and the releasing of energy from the device into the targeted heart region.

While the methods and apparatus of the present description are described with particular reference to electrical energy delivery, most particular radiofrequency and/or microwave energy, it will be appreciated that many aspects of the description apply equally to other energy and ablative sources, such as vibrational mechanical energy, e.g., ultrasound; optical energy, e.g., laser; cryogenic delivery; chemical agent delivery, and the like. The present invention is set out in the appended claims. The embodiments, aspects or examples according to the present description that do not fall within the scope of said claims are provided for illustrative purposes only and do not form part of the present invention.

### BRIEF DESCRIPTION OF THE DRAWINGS

Figure 1 shows one wire tip ablation catheter embodiment.
Figure 2 shows one embodiment of the wire tip ablation catheter.
Figure 3 shows one embodiment of the wire tip ablation catheter utilizing conductive plates.
Figure 4 shows one embodiment of the wire tip ablation catheter utilizing conductive coils.
Figure 5 shows one embodiment of the umbrella tip ablation catheter.
Figure 6 shows one embodiment of the umbrella tip ablation catheter.
Figure 7 shows one embodiment of the umbrella tip ablation catheter.
Figure 8 shows one embodiment of the umbrella tip ablation catheter.
Figure 9 shows one embodiment of the umbrella tip ablation catheter.
Figure 10 shows one embodiment of the umbrella tip ablation catheter.
Figure 11 shows one embodiment of the umbrella tip ablation catheter.

### DETAILED DESCRIPTION OF THE INVENTION

The present description provides catheters for performing targeted tissue ablation in a subject. In preferred embodiments, the catheters comprise a catheter body having a proximal end and distal end and usually a lumen therebetween. The catheter is preferably of the type used for performing intracardiac procedures, typically being introduced from the femoral vein in a patient's leg. The catheter is preferably introducable through a sheath and preferably has a steerable tip, as described generally above, which allows positioning of the distal tip when the distal end of the catheter is within a heart chamber. The catheters preferably include electrode arrays which are deployable from the distal end of the catheter to engage a plurality of individual electrode elements within the array against cardiac tissue, typically atrial wall tissue or other endocardial tissue. Electrode arrays may be configured in a wide variety of ways. In particular, the present description provides devices comprising wire tipped and umbrella tipped catheter ablation devices, and methods for treating conditions (*e.g*., atrial fibrillation, supra ventricular tachycardia, atrial tachycardia, ventricular tachycardia, ventricular fibrillation, and the like with these devices.

As described above, the normal functioning of the heart relies on proper electrical impulse generation and transmission. In certain heart diseases (*e.g*., atrial fibrillation) proper electrical generation and transmission are disrupted. In order to restore proper electrical impulse generation and transmission, the catheters of the present description may be employed.

In general, catheter ablation therapy provides a method of treating cardiac arrhythmias. Physicians make use of catheters to gain access into interior regions of the body. Catheters with attached electrode arrays or other ablating devices are used to destroy targeted cardiac tissue. In the treatment of cardiac arrhythmias, a specific area of cardiac tissue having aberrant conductive pathways, such as atrial rotors, emitting or conducting erratic electrical impulses is initially localized. A user (*e.g*., a physician) directs a catheter through a main vein or artery into the interior region of the heart that is to be treated. The ablating element is next placed near the targeted cardiac tissue that is to be ablated. The physician directs an energy source from the ablating element to ablate the tissue and form a lesion. In general, the goal of catheter ablation therapy is to destroy cardiac tissue suspected of emitting erratic electric impulses, thereby curing the heart of the disorder. For treatment of atrial fibrillation currently available methods have shown only limited success and/or employ devices that are not practical.

The ablation catheters of the present description allow the generation of lesions of appropriate size and shape to treat conditions involving disorganized electrical conduction (*e.g*., atrial fibrillation). The ablation catheters of the present description are also practical in terms of ease-of-use and risk to the patient. Previous catheters that generate linear or curvilinear lesion in the left or right atrial tissue may have limited efficacy. Moreover, the procedure times and the incidence of complications were significantly high with these approaches. Another approach utilizes encircling of the left atrial tissue by point-by-point applications of radio-frequency energy. However to generate complete circles this approach can be time-consuming and may have a limited efficacy. The present description addresses this need with, for example, wire tip and umbrella ablation catheters and methods of using these ablation catheters to create spiral or radial lesions in the endocardial surface of the atria by delivery of energy (*e.g.*, radio-frequency). The lesions created by the wire tipped and umbrella tipped ablation catheters are suitable for inhibiting the propagation of inappropriate electrical impulses in the heart for prevention of reentrant arrhythmias.

Definitions. To facilitate an understanding of the invention, a number of terms are defined below.

As used herein, the terms "subject" and "patient" refer to any animal, such as a mammal like livestock, pets, and preferably a human. Specific examples of "subjects" and "patients" include, but are not limited, to individuals requiring medical assistance, and in particular, requiring atrial fibrillation catheter ablation treatment.

As used herein, the terms "catheter ablation" or "ablation procedures" or "ablation therapy," and like terms, refer to what is generally known as tissue destruction procedures. Ablation is often used in treating several medical conditions, including abnormal heart rhythms. It can be performed both surgically and non-surgically. Non-surgical ablation is typically performed in a special lab called the electrophysiology (EP) laboratory. During this non-surgical procedure a catheter is inserted into the heart and then a special machine is used to direct energy to the heart muscle. This energy either "disconnects" or "isolates" the pathway of the abnormal rhythm (depending on the type of ablation). It can also be used to disconnect the conductive pathway between the upper chambers (atria) and the lower chambers (ventricles) of the heart. For individuals requiring heart surgery, ablation can be performed during coronary artery bypass or valve surgery.

As used herein, the term "wire tip body" refers to the distal most portion of a wire tip catheter ablation instrument. A wire tip body is not limited to any particular size. A wire tip body may be configured for energy emission during an ablation procedure.

As used herein, the term "spiral tip" refers to a wire tip body configured into the shape of a spiral. The spiral tip is not limited in the number of spirals it may contain. Examples include, but are not limited to, a wire tip body with one spiral, two spirals, ten spirals, and a half of a spiral.

As used herein the term "umbrella tip body" refers to the distal most portion of an umbrella tip catheter ablation instrument. An umbrella tip body is not limited to any particular size. An umbrella tip body may be configured for energy emission during an ablation procedure.

As used herein, the term "lesion," or "ablation lesion," and like terms, refers to tissue that has received ablation therapy. Examples include, but are not limited to, scars, scabs, dead tissue, and burned tissue.

As used herein, the term "spiral lesion" refers to an ablation lesion delivered through a wire tip ablation catheter. Examples include, but are not limited to, lesions in the shape of a wide spiral, and a narrow spiral.

As used herein, the term "umbrella lesion" or "radial lesion," and like terms, refers to an ablation lesion delivered through an umbrella tip ablation catheter. Examples include, but are not limited to, lesions with five equilateral prongs extending from center point, lesions with four equilateral prongs extending from center point, lesions with three equilateral prongs extending from center point, and lesions with five non-equilateral prongs extending from center point.

As used herein, the term "conductive coil" refers to electrodes capable of emitting energy from an energy source in the shape of a coil. A conductive coil is not limited to any particular size or measurement. Examples include, but are not limited to, densely wound copper, densely wound platinum, and loosely wound silver.

As used herein, the term "conductive plate" refers to electrodes capable of emitting energy from an energy source in the shape of a plate. A conductive plate is not limited to any particular size or measurement. Examples include, but are not limited to, copper plates, silver plates, and platinum plates.

As used herein, the term "energy" or "energy source," and like terms, refers to the type of energy utilized in ablation procedures. Examples include, but are not limited to, radio-frequency energy, microwave energy, cryo energy (*e.g*., liquid nitrogen), or ultrasound energy, laser (optical) energy, mechanical energy, and the like.

As used herein, the term "maze procedure," "maze technique," "maze ablation," and like terms, refer to what is generally known as a cardiac ablation technique. Small lesions are made at a specific location in the heart in a manner so as to create a "maze." The maze is expected to prevent propagation of electrical impulses.

As used herein, the term "central post" refers to a chamber capable of housing small items. The central post is made from a durable material. A central post is not limited to any particular size or measurement. Examples include, but are not limited to, polyurethane, steel, titanium, and polyethylene.

As used herein, the term "outer arms" refers to a shaft capable of interfacing with electrodes and a central post. An outer arm is not limited to any size or measurement. Examples include, but are not limited, to titanium shafts, polyurethane shafts, and steel shafts.

As used herein, the term "outer arm hinge" refers to a joint (*e.g.*, junction, flexion point) located on an outer arm. The degree of flexion for an outer arm hinge may range from 0 to 360 degrees.

The present description provides structures that embody aspects of the ablation catheter. The present description also provides tissue ablation systems and methods for using such ablation systems. The illustrated and preferred embodiments discuss these structures and techniques in the context of catheter-based cardiac ablation. These structures, systems, and techniques are well suited for use in the field of cardiac ablation.

However, it should be appreciated that the description is applicable for use in other tissue ablation applications. For example, the various aspects of the description have application in procedures for ablating tissue in the prostrate, brain, gall bladder, uterus, and other regions of the body, using systems that are not necessarily catheter-based.

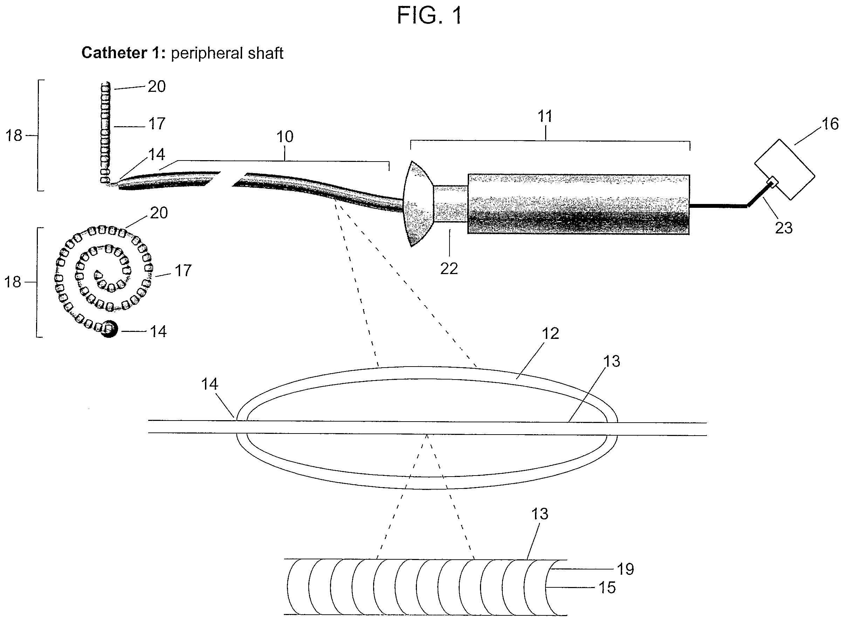

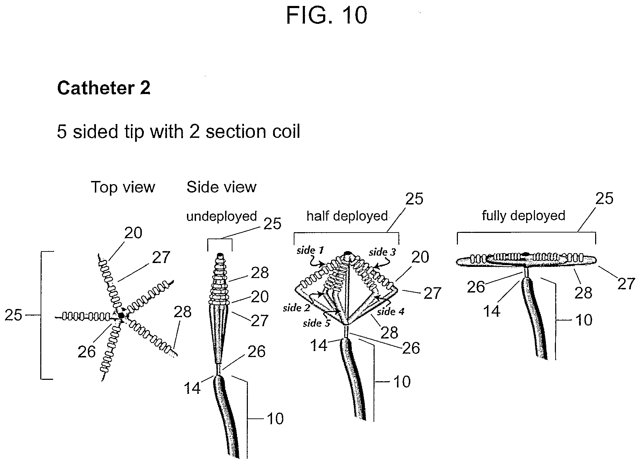

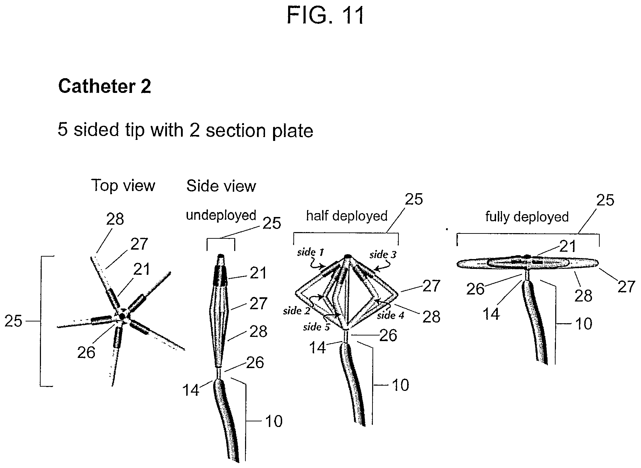

The multifunctional catheters of the present description have advantages over previous prior art devices. FIGS. 1-11 show various preferred embodiments of the multifunctional catheters of the present description. The present description is not limited to these particular configurations.

Wire Tip Ablation Catheters. FIG. 1 illustrates an ablation catheter embodiment including broadly an elongate catheter body 10 (*e.g*., hollow tube) extending from a handle 11. Elongate catheter body 10 permits the housing of items that assist in the ablation of subject tissue (*e.g*., human tissue and other animal tissue, such as cows, pigs, cats, dogs, or any other mammal). The elongate catheter body 10 may range in size so long as it is not so small that it cannot carry necessary ablation items, and not so large so that it may not fit in a peripheral major vein or artery. The elongate catheter body 10 includes an elongate sheath 12 (*e.g*., protective covering). The elongate sheath 12 may be made of a polymeric, electrically nonconductive material, like polyethylene or polyurethane. In preferred embodiments, the elongate sheath 12 is formed with the nylon based plastic Pbax, which is braided for strength and stability. In additional embodiments, the elongate sheath 12 is formed with hypo tubing *(e.g.,* stainless steel, titanium). The elongate sheath 12 houses a conducting wire 13 *(e.g.,* standard electrical wire) and a thermal monitoring circuit 19. The conducting wire extends from the handle 11 through the distal opening 14. In addition, the conducting wire 13 is wrapped with a steering spring 15. The conducting wire 13 is flexible so that it may be flexed to assume various positions (*e.g*., curvilinear positions). The steering spring 15 is controlled through manipulation of the handle 11, as discussed below. The conducting wire 13 is also capable of transmitting energy (*e.g.*, radio-frequency energy) from an energy source 16 (*e.g*., radio-frequency energy generator).

A thermal monitoring circuit 19 (*e.g*., thermocouple) is coupled with the conducting wire 13 and extends from the handle 11 through the umbrella tip body 25. The thermal monitoring circuit 19 connects with energy source cable 23 within handle 11. Regulation of the thermal monitoring circuit 19 is achieved through the energy source 16. In some embodiments, the present description utilizes the thermal monitoring circuit described in U.S. 6,425,894. whereby a thermocouple is comprised of a plurality of thermal monitoring circuits joined in series. The thermal monitoring circuits are thermoconductively coupled to the electrodes. In some embodiments, the thermal monitoring circuit employs two wires to travel through the elongated catheter body in order to monitor a plurality of electrodes.

The distal opening 14 is the distal terminus of the elongate catheter body 10. At the distal opening 14, the conducting wire 13 exits the elongate sheath 12. While the majority of the conducting wire 13 is housed within the elongate sheath 12, the distal portion is housed within the wire tip sheath 17. The wire tip sheath 17 begins at the distal opening 14 and extends throughout the wire tip body 18. The wire tip sheath 17 may be made of a polymeric; electrically nonconductive material (*e.g*., polyethylene or polyurethane). In preferred embodiments, the wire tip sheath 17 is formed with peek insulator (*e.g*., high temperature thermo-plastic). A thermal monitoring circuit 19 is coupled with the conducting wire 13 and extends from the handle 11 through the wire tip body 18. The thermal monitoring circuit 19 connects with energy source cable 23 within handle 11.

The wire tip sheath 17 permits the wire tip body 18 to be molded or shaped into a desired position. In preferred embodiments, the wire tip body 18 may be shaped into a unique shape (*e.g*., spiral).

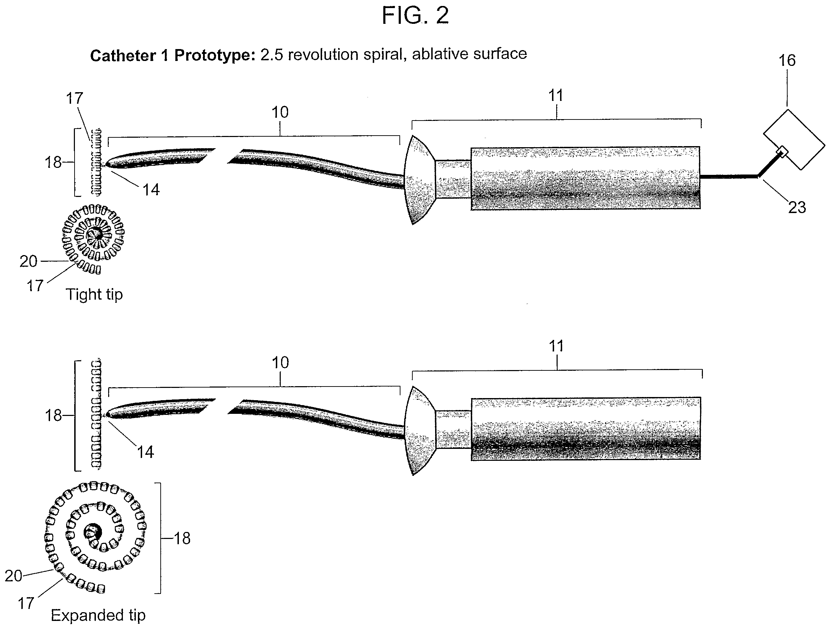

In the preferred embodiment described FIGS. 1-4, the wire tip body 18 is in the shape of a spiral which is the support for the electrode array. The spiral on a wire tip body 18 may be peripheral to or central to the elongate catheter body 10. The spiral wire tip body 18 is central if the spiral interfaces with the distal opening 14 at the spiral center point, and peripheral if the spiral interfaces with the distal opening 14 at the spiral exterior point. The embodiment described in FIG. 1 presents a spiral wire tip body 18 that is peripheral to the elongate catheter body 10. Alternatively, the embodiment described in FIG. 2 presents a spiral wire tip body 18 that is central to the elongate catheter body. A wire tip body 18 in the shape of a spiral may comprise any number of complete rotations (*e.g.*, complete spirals). In the embodiment described in FIGS. 1 and 2, the spiral wire tip body 18 consists of two and one half complete rotations.

The spiral wire tip body 18 is illustrated as laying at an angle of about 90° relative to the axis of the catheter body. While this is the preferred orientation, the tip body 18 could lie at an angle up to 45° relative to the axis. Usually, a hinge of other connection between the tip body 18 and the supporting structure will be sufficiently flexible to allow the body to conform to the endocardial surface as the catheter is advanced against that surface. Such geometries would be useful for accessing particular regions of an atrial or other endocardial wall.

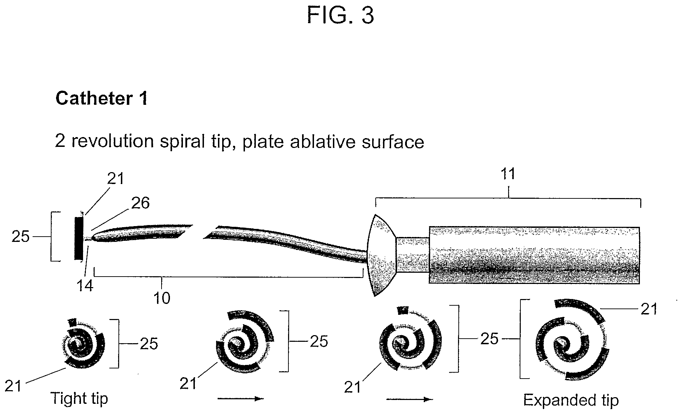

Alternatively, the embodiment described in FIG. 3 presents a spiral with only two complete rotations. The distance between the spirals on the wire tip body 18 may assume any measurement.

Tissue ablation occurs on the wire tip body 18. Various conductive elements (*e.g*., coils or plates) may be distributed along the wire tip body 18 to form an electrode array. The energy utilized within a catheter ablation instrument is released through the conductive elements. The number of conductive elements on the wire tip body 18 permit a determined energy release and resulting ablation lesion.

The conductive electrode elements 2 and 4 used in the preferred embodiment described in FIG. 1, are conductive coils 20. Each conductive coil 20 is an electrode that is comprised of a densely wound continuous ring of conductive material, (*e.g*., silver, copper). In preferred embodiments, the conductive coil 20 is made from platinum. The conductive coils 20 are fitted (*e.g*., pressure fitting) about the wire tip body 18. In preferred embodiments, a conductive coil 20 is soldered onto a conductive metal (*e.g*., copper, copper with silver) and swaged onto the wire tip body 18. Additional embodiments may utilize an adhesive seal in addition to swaging in fixing conductive coils 20 to the wire tip body 18. A conductive coil 20 may range in size from 0.1 mm to 20 mm. In preferred embodiments, a conductive coil 20 ranges in size from 2 to 8 mm. The conductive coils 20 interact with the conducting wire 13 and emit the energy carried by the conductive wire 13.

Conductive coils 20 may be arranged in many different patterns (*e.g*., staggered) along the wire tip body 18. Usually, the electrode array patterns may involve repeating sets of conductive coils 20 *(e.g.,* set of 3 coils - 3 coils - 3 coils, etc.) or nonrepeating sets (*e.g*., set of 3 coils - 5 coils - 2 coils, etc.). The pattern of conductive coils 20 arranged in the preferred embodiment presented in FIGS. 1, 2 and 4 consist of a repeating set of four conductive coils 20 separated by a non-conductive gap. In general, the coils may have lengths in the range from 2 mm to 20 mm and the gap may range in size from 1 mm to 10 mm, and is nonconductive. In the embodiments demonstrated in FIGS. 1, 2 and 4, the gap size is 5 mm. In some embodiments, there may be no gap between electrode elements, but such a design is not preferred.

The conductive elements used in the preferred embodiment described in FIG. 3 are conductive plates 21. Each conductive plate 21 is an electrode that is comprised of a solid ring of conductive material (*e.g*., platinum). The conductive plates 21 are fitted (*e.g*., pressure fitting) about the wire tip body 18. Additional embodiments may utilize an adhesive seal in addition to swaging in fixing conductive plates 21 to the wire tip body 18. A conductive plate 21 may range in size from 2 mm to 20 mm. The conductive plates 21 interact with the conducting wire 13 and emit the energy carried by the conductive wire 13.

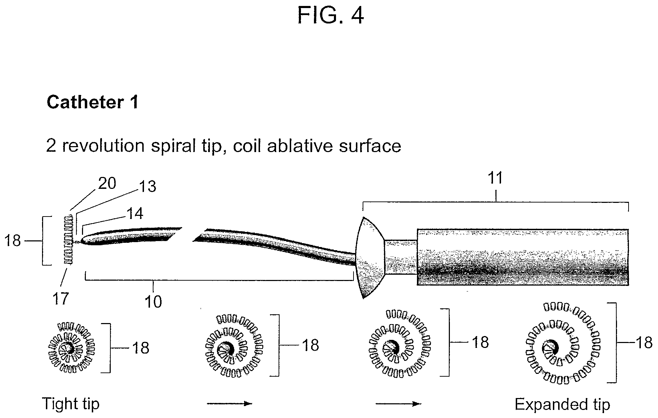

Conductive plates 21 may be arranged in many different patterns (*e.g*., repeating sets) along the wire tip body 18. Such patterns may involve a repeating series of conductive plates 21 separated by spaces (*e.g*., plate - space - plate - space - plate; etc.) or a random series (*e.g*., space - space - plate - plate - plate - space - plate; etc.). In addition, the pattern of conductive plates 21 may simply involve only one short or extended conductive plate 19. The pattern arranged in the preferred embodiment presented in FIG. 3 consists of four conductive plates 21 separated by nonconductive gaps. In general, the gaps may range in size from 1 mm to 10 mm. In the FIG. 4 embodiment, the gap size is 5 mm.

The pattern of conductive elements arranged on the wire tip body 18 need not be restricted to only a certain type. Indeed, the present description envisions a wire tip body 18 with varied patterns of different conductive elements (*e.g*., coil - gap - plate - plate - gap - coil - coil; etc.).

The wire tip body 18 may be expanded or contracted through manipulation of the handle 11. In preferred embodiments, the handle 11 connects with the conducting wire 13 with the steering spring 15 attached onto it. The conducting wire 13 attaches onto a lever 22 inside the handle 11. Extension of the lever 22 causes a contraction in the steering spring 15 attached to the conducting wire 13 resulting in a constricting of the wire tip body 18. Alternatively, constriction of the lever 22 causes the steering spring 15 to expand.

An alternative embodiment utilizes the steering method described in U.S. 5,318,525. In that embodiment, a catheter tip is deflected by means of a shapable handle coupled to pull wires fastened to the distal end of the deflectable tip. A core wire extends from the handle to the distal tip, providing fine positioning of the deflectable tip by applying torque through the core wire to the tip. A spring tube is further provided in the deflectable tip for improved torque transmission and kink-resistance. The catheter has an electrode at the distal end of the deflectable tip for positioning at a target site and applying RF power to accomplish ablation.

In other embodiments, the method of catheter manipulation described in U.S. 2001/0044625 A1 is utilized, whereby a control element within the handle is able to flex and deflex the distal tip. Additional embodiments utilize the method of catheter manipulation described in U.S. 6,241,728, whereby three handle manipulators permit a distal tip to be deflected longitudinally, radially, and in a torqued position. A further embodiment utilizes the method of catheter manipulation described in U.S. 2001/0029366 A1, whereby a rotating cam wheel permits the steering of a distal tip in any direction. However, other mechanisms for steering or deflecting the distal end of a catheter according to the present description may also be employed. For example, the steering and deflection mechanism as set forth in U.S. 5,487,757 may also be employed to deflect the distal tip of the catheter, as well as any other known deflection/steering mechanism. Similarly, a sliding core wire for adjustment of the radius of curvature of the catheter when deflected may also be employed, as also disclosed in U.S. Pat. No. 5,487,757.

In alternative embodiments, the wire tip body 18 may be expanded or contracted though computer assisted manipulation. In other embodiments, the wire tip body 18 may be manipulated through use of magnetic fields.

The terminus of the conducting wire attaches to an energy source cable 23 that establishes a connection with the energy source 16.

Depictions of various degrees of contraction or expansion of the wire tip body 18 in the shape of a spiral are presented in FIGS. 2, 3 and 4. In the fully contracted position, the regions between the spirals on the wire tip body 18 decreases while the spacing in between the conductive elements remains intact. As the wire tip body 18 becomes more expanded, the regions in between spirals on the wire tip body 18 increases, and the spacing in between the conductive elements remains intact.

The proximal origin of the conducting wire 13 may be located at the distal end of the handle 11. At the proximal origin of the conducting wire 13, the conducting wire 13 is connected with an energy source 16 (*e.g*., radio-frequency energy or microwave). Embodiments of the present description may utilize numerous forms of energy (*e.g*., radio-frequency energy, liquid nitrogen, saline). In one embodiment, liquid nitrogen is utilized as an energy source (this requires a different design: a hollow tube that traveks throughout the catheter to deliver N2 gas) 16 that freezes a particular tissue region. In an additional embodiment, the energy source 16 utilized is a saline irrigation system, whereby saline is flushed out through a mesh of electrodes carrying an electric current.

In preferred embodiments, radio-frequency energy or microwave is utilized as the energy source 16. Various radio-frequency energy and microwave generators are commercially available. For radio-frequency protocols, the source 16 is usually operated in a unipolar manner where a large (20 x 10 cm) ground patch 24 is attached to the patient's back to complete the circuit. The current travels from the tip of the heart to the patch. The amount of energy utilized may be controlled by adjusting the power output of the energy source 16. Four parameters may are regulated through the energy source 16: power output, impedance, temperature, and duration of energy application. Optionally, the same source 16 could be modified to provide for bipolar energy application as well as unipolar and bipolar mapping.

The precise pattern of conductive elements assorted on the wire tip body 18 along with the shaped configuration of the wire tip body 18 permits a unique type of ablation lesion ranging from long and thin to large and deep in shape. In addition, numerous types of ablation lesions are possible for each catheter ablator embodiment through manipulation of the wire tip body 18.

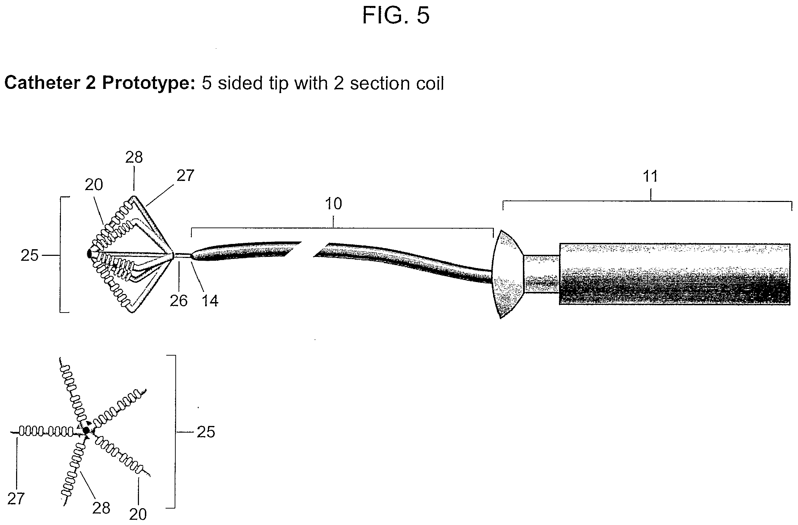

Umbrella Tip Ablation Catheters FIGS. 5-11 illustrate ablation catheter embodiments including broadly an elongate catheter body 10 (*e.g*., hollow tube) extending from a handle 11. The elongate catheter body 10 includes an elongate sheath 12 *(e.g.,* protective covering). The elongate sheath 12 houses a conducting wire 13 (*e.g*., standard electrical wire) and a thermal monitoring circuit 19. The conducting wire extends from the handle 11 through the distal opening 14. The conducting wire 13 is also capable of transmitting energy *(e.g.,* radio-frequency energy) from an energy source 16 *(e.g.,* radio-frequency energy generator).

A thermal monitoring circuit 19 (*e.g*., thermocouple) may be coupled with the conducting wire 13 and extend from the handle 11 through the umbrella tip body 25. The thermal monitoring circuit 19 connects with energy source cable 23 within handle 11. Regulation of the thermal monitoring circuit 19 is achieved through the energy source 16. In some embodiments, the present description utilizes the thermal monitoring circuit described in U.S. 6,425,894 whereby a thermocouple is comprised of a plurality of thermal monitoring circuits joined in series. The thermal monitoring circuits thermoconductively coupled to the electrodes. The thermal monitoring circuit will require only two wires to travel through the elongated catheter body in order to monitor a plurality of electrodes.

The distal opening 14 is the distal terminus of the elongate catheter body 10. The most distal portion of this embodiment is the umbrella tip body 25. The umbrella tip body 25 consists of a central post 26, a plurality of outer arms 27, the conductive wire 13, and conductive elements (*e.g*., coils).

The central post 26 extends from the distal opening 14. The central post 26 is typically tubular with a lumen adapted to receive a guidewire. The central post 26 may be made from electrically nonconductive materials (*e.g*., polyurethane, plastic, or polyethylene). The length of the central post 26 may range from 0.1 mm to 100 mm, and its diameter from 0.001 mm to 100 mm. The central post 26 maybe formed into numerous shapes. In the preferred embodiments described in FIGS. 5-11, the central post 26 is in the shape of an extended cylindrical rod.

One function of the central post 26 is to house the conducting wire 13. At the distal opening 14, the conducting wire 13 exits the elongate sheath 12. While the majority of the conducting wire 13 is housed within the elongate sheath 12, the distal portion is housed within the central post 26.

The outer arms 27 extend from the base of the central post 26 through the top of the central post 27. An outer arm 27 is a shaft *(e.g.,* post) made from an electrically nonconductive material (*e.g*., polyurethane, polyethylene). The length of an outer arm 27 may range from 0.1 mm to 100 mm, and its diameter from 0.001 mm to 100 mm. In some embodiments, along the outside of an outer arm 27 is a thermal monitoring circuit 19, which is able to detect temperature and maintain temperature.

An outer arm 27 may be flexible or rigid. In the preferred embodiments described in FIGS. 5-11, the outer arms 27 are flexible. The degree of flexibility may range from 0 to 360 degrees. There are several types of outer arm 27 flexibility. The outer arm 27 flexibility displayed in FIGS. 5-11 arises from an outer arm hinge 28 located at the outer arm's 27 midpoint and permits a degree of flexibility from 0 to 180 degrees.

One function of the outer arms 27 is to interact with the central post 26. The central post 26 and each outer arm 27 firmly connect (*e.g*., adhere) at the top of the central post 26. The outer arms 27 also interface (*e.g.*, connect) at the base of the central post 26. The outer arm 27 connections at the base of the central post 26 may or may not also connect with the central post 27. In the preferred embodiments described in FIGS. 5-11, the outer arms 27 interface together at the distal opening 14 at a distal opening ring 29. The distal opening ring 29 does not connect to the central post 26, but rather connects to the distal opening 14.

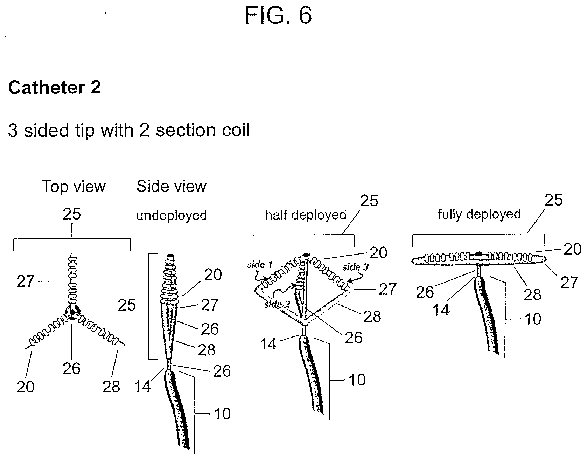

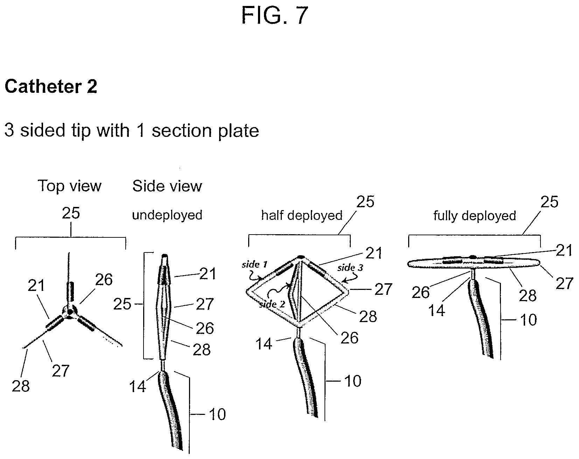

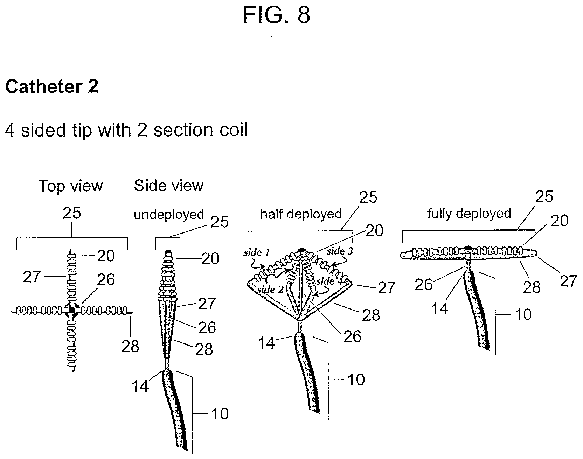

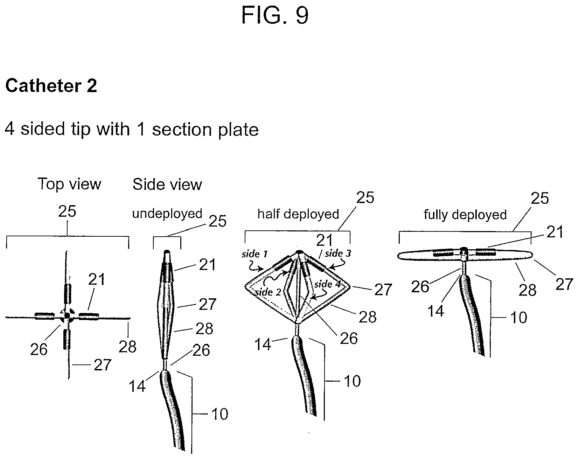

Umbrella tip bodies 26 may present a plurality of outer arms 27. The embodiments described in FIGS. 5, 10 and 11 display an umbrella tip 26 with five outer arms 27. The embodiments described in FIGS. 6 and 7 display an umbrella tip body 26 with three outer arms 27. The embodiments described in FIGS. 8 and 9 display an umbrella tip body 26 with four outer arms 27. There may be any range of distances in between each outer arm 27 on an umbrella tip 26. In the embodiments displayed in FIGS. 5-11 the distances in between each outer arm 27 are equilateral.

Conductive elements (*e.g*., plates) are distributed along the outer arms 27. The energy utilized within a catheter ablation instrument is released through the conductive elements. The number of conductive elements an outer arm 27 permits a determined energy release and resulting ablation lesion.

The conductive elements used in the preferred embodiments described in FIGS. 5, 6, 8, and 10 are conductive coils 20. Each conductive coil 20 is an electrode that is comprised of a densely wound continuous ring of conductive material, (*e.g*., silver, copper). In preferred embodiments, the conductive coil 20 is made from platinum. The conductive coils 20 are fitted (*e.g*., pressure fitting) about the wire tip body 18. In preferred embodiments, a conductive coil 20 is soldered onto a conductive metal (*e.g*., copper, copper with silver) and swaged onto the umbrella tip body 25. Additional embodiments may utilize an adhesive seal in addition to swaging in fixing conductive coils 20 to the umbrella tip body 25. A conductive coil 20 may range in size from 0.1 mm to 20 mm. The conductive coils 20 interact with the conducting wire 13 and emit the energy carried by the conductive wire 13.

Conductive coils 20 may be arranged in many different patterns (*e.g*., staggered) along an outer arm 27. Such patterns may involve repeating sets of conductive coils 20 (*e.g.,* set of 3 coils - 3 coils - 3 coils, etc.) or nonrepeating sets (*e.g.,* set of 3 coils - 5 coils - 2 coils, etc.). In addition, an umbrella tip body 26 may vary the patterns of conductive coils 20 on each outer arm 27 to achieve an even more unique ablation lesion. The pattern of conductive coils 20 arranged in the preferred embodiment presented in FIGS. 5, 6, 8, and 10 consist of two sets of four conductive coils 20 separated by a gap on each outer arm 27 located near the distal ending. In general, the gaps may range in size as described above and are nonconductive.

The conductive elements used in the preferred embodiment described in FIG. 7, 9, and 11 are conductive plates 21. Each conductive plate 21 is an electrode that is comprised of a solid ring of conductive material, (*e.g*., platinum). The conductive plates 21 are fitted (*e.g*., pressure fitting) about an outer arm 27. A conductive plate 21 may range in size from 0.1 mm to 20 mm. The conductive plates 19 interact with the conducting wire 13 and emit the energy carried by the conductive wire 13.

Conductive plates 21 may be arranged in many different patterns (*e.g*., repeating sets) along an outer arm 27. Such patterns may involve a repeating series of conductive plates 21 separated by spaces (*e.g*., plate - space - plate - space - plate; etc.) or a random series (*e.g*., space - space - plate - plate - plate - space - plate; etc.). The pattern of conductive plates 21 may simply involve only one short or extended conductive plate 21. In addition, an umbrella tip body 26 may vary the patterns of conductive plates 21 on each outer arm 27 to achieve an even more unique ablation lesion. The pattern arranged in the preferred embodiment presented in FIG. 7, 9, and 11 consists of one conductive plates 21 on each outer arm 27 located near the distal ending.

The pattern of conductive elements arranged on the umbrella tip body 26 need not be restricted to only a certain type. Indeed, the present description contemplates an umbrella tip 26 with varied patterns of different conductive elements (*e.g*., outer arm 1: coil - plate - plate - coil; outer arm 2: plate - plate - coil; outer arm 3: coil - coil; etc.).

An umbrella tip 26 may be expanded or contracted through manipulation of the handle 11. In one type of embodiment, the base of the central post 26 interfaces (*e.g*., adheres) with the conducting wire 14. The distal opening 14 is wide enough for the central post 26 to slide in and out of the elongate catheter body 10. Contraction of the umbrella tip 26 occurs when the central post 26 is extended out of the elongate catheter body 10.

Expansion of the umbrella tip 26 occurs when the central post 26 is extended into the elongate catheter body 10.

Extension or retraction of the umbrella tip body 26 is manipulated through the handle 11. In preferred embodiments, the handle 11 connects with the conducting wire 13 and steering spring 15. The conducting wire 13 attaches onto a lever 22 inside the handle 11. Extension of the lever 22 causes the central post 26 to extend outside of the elongate catheter body 10. As the central post 26 extends outside the elongate catheter body 10, the outer arms 27 reduce the degree of flexion. Retraction of the lever 22 causes the central post 26 to withdraw inside the elongate catheter body 10. As the central post 26 withdraws into the elongate catheter body 10, the outer arms 27 increase the degree of flexion.

An umbrella tip catheter may utilize numerous alternative steering embodiments, some of which are described above in relation to wire tip ablation catheters.

The terminus of the conducting wire attaches to an energy source cable 23 which establishes a connection with the energy source 16.

The proximal origin of the conducting wire 13 may be located at the distal end of the handle 11. At the proximal origin of the conducting wire 13, the conducting wire 13 is connected with an energy source 16. Embodiments of the present description may utilize numerous forms of energy (*e.g*., radio-frequency energy, utrasound, laser, liquid nitrogen, saline-mediated).

In preferred embodiments, radio-frequency energy is utilized as the energy source 16. Various radio-frequency energy generators are commercially available. A large (20 x 10 cm) ground patch 24 is attached to the patient's back to complete the circuit. The current travels from the tip of the heart to the patch. The amount of energy utilized may be controlled by adjusting the power output of the energy source 16. Four parameters may are regulated through the energy source 16: power output, impedance, temperature, and duration of energy application.

The precise pattern of conductive elements assorted on an umbrella tip 26, along with the varying degrees of central post 26 expansion or contraction, permits a unique type of ablation lesion ranging from long and thin to large and deep in shape.

In the preferred embodiments, the electrodes arrays on the catheters may also be used for mapping or detecting the location of the aberrant conductive pathways in the atrial or other endocardial wall. The individual electrode elements, such as the coils in either the spiral or umbrella catheter embodiments as described above, are electrically isolated and separately connectable to external electrical mapping circuitry. Bipolar recordings may then be made between any pairs or combinations of the electrode elements. Unipolar mapping may be preferred between the electrode element(s) and another indifferent or dispersing electrode(s) in a location remote from the heart chamber surface, typically an external surface or in a great vessel such as the inferior vena cava. Units comprising such mapping circuitry are commercially available and well-described in both the patent and medical literature. The electrodes will generally be connected in a bipolar manner where electrical potentials between any two or more of the electrically isolated electrode elements can be measured but may also be connected in a unipolar pattern. Based on these differences in electrical potential, the aberrant conductive pathways can be detected using conventional techniques.

Alternative Embodiments The present description is not limited to wire tip or umbrella tip embodiments. It is contemplated that fragmented ablation lesions may be created with alternative designs. For example, zig-zag distal bodies, cross-hatch patterns, or other shapes may be utilized so long as the ablation lesion that is created is effective in prevention propagation electrical impulses.

Uses: The multifunctional catheter of the present description has many advantages over the prior art. The heart has four chambers, or areas. During each heartbeat, the two upper chambers (atria) contract, followed by the two lower chambers (ventricles). A heart beats in a constant rhythm -- about 60 to 100 times per minute at rest. This action is directed by the heart's electrical system. An electrical impulse begins in an area called the sinus node, located in the upper part of the right atrium. When the sinus node fires, an impulse of electrical activity spreads through the right and left atria causing them to contract, forcing blood into the ventricles. Then the electrical impulses travel in an orderly manner to another area called the atrioventricular (AV) node and HIS-Purkinje network. The AV node is the electrical bridge that allows the impulse to go from the atria to the ventricles. The HIS-Purkinje network carries the impulses throughout the ventricles. The impulse then travels through the walls of the ventricle, causing them to contract. This forces blood out of the heart to the lungs and the body. Each electrical circuit has a wavelength. The wavelength is equivalent to the product of the impulse's conduction velocity and the impulse's effective refractory period.

Atrial fibrillation is the most common type of irregular heartbeat. In atrial fibrillation, an electrical impulse does not travel in an orderly fashion through the atria. Instead, many impulses begin and spread through the atria and compete for a chance to travel through the AV node. Such aberrant electrical impulses may originate from tissues other than the heart's electrical system.

One method of treatment for atrial fibrillation is ablation therapy. It is estimated that for initiation of atrial fibrillation, premature depolarizations from any cardiac structure is necessary. However, for perpetuation of atrial fibrillation both a continuous/continual surge of premature depolarizations and an atrial substrate capable of maintaining multiple reentrant circuits of atrial fibrillation are necessary. The goal of ablation therapy is to eliminate the premature depolarizations that trigger atrial fibrillation, and also to modify the atrial tissue such that the minimum wavelength of a reentrant electrical circuit will not be able to fit into the atrial tissue. Procedurally, to eliminate triggers, a specific and localized area of interest (*e.g*., area of pulmonary vein connecting with atria, alternate group of cells emitting electrical impulses on their own) is targeted. A catheter with an ablation instrument is directed through a major vein or artery to the targeted location in the left atrium. Through the ablation instrument, radio-frequency is released onto the targeted location. A resulting scar or lesion is created. To modify the latrial substrate certain "maze" patterns of ablation lesions are created. The intent has been to create continuous lesions without any connecting gaps.

The major shortcoming of present ablation techniques is an inability to avoid gaps in the maze ablation process. The heart walls have extremely complex curvatures making the creation of a continuous ablation maze nearly impossible. The typical result is an ablation maze containing numerous gaps. It is important to avoid the presence of gaps within the ablation maze because aberrant electrical impulses are able to propagate through them resulting in secondary arrhythmias. As such, gaps become reentrant circuits, and the atrial fibrillation is capable of continuing and different arrhythmias such as atrial flutter may also occur. In addition creation of maze like lesions in atrium is extremely time consuming and is associated with a significant complication rate.

The present multifunctional catheter overcomes the gap problem faced in the prior art by not relying upon continuous lesions. The present invention creates spiral or umbrella shaped ablation lesions with very small gaps between the ablation lesions. Each gap is not large enough to allow an electrical impulse to propagate through it. The ablation tips of the present invention *(e.g.,* wire tip or umbrella tip) have a relatively small surface area *(e.g.,* 10-25 mm in diameter). In addition, the tips are pliable and soft, and yet have good support form the shaft. Thus, when the tip is pushed against the atrial wall, most, if not all, of the surface will form good contact without the risk of perforation as it is not a pointed catheter tip. Strategic placement of such ablation lesions essentially decreases the effective atrial mass that an aberrant electrical impulse may propagate through. This represents a significant improvement over the prior art because no longer will the laborious and often unsuccessful creation of ablation lesion mazes be necessary. It is also possible to use the ablation approach described in this disclosure in conjunction with ablation strategies that target elimination of triggers such as a pulmonary vein isolation procedure.

The present ablation catheters may be utilized in treating cardiac disorders including those listed above. In addition, the present ablation catheter may be utilized in several other medical treatments (*e.g*., ablation of solid tumors, destruction of tissues, assistance in surgical procedures, kidney stone removal).

The invention is set out in the claims that follow:

## Claims

1. A device for high density mapping and for tissue ablation comprising:
- an elongate catheter body (10) having a proximal end and a distal end;
- a spiral tip or an umbrella tip (25) having a plurality of outer arms (27) arranged to open radially from a common point outside the catheter body, wherein said spiral tip or umbrella tip (25) is deployable from the distal end of the catheter body (10)
- conductive elements (20, 21) disposed on the spiral tip or on the outer arms (27) of the umbrella tip (25)and configured to map and ablate tissue;
wherein the spiral tip or umbrella tip comprises from 6 to 30, conductive elements (20, 21) distributed over an area in the range from 1 cm² to 12 cm² and wherein said conductive elements (20, 21) have a maximum length or other dimension in the range from 2 mm to 20 mm, and are spaced-apart by a distance in the range from 1 mm to 10 mm; and
- a handle (11) disposed at the proximal end of the elongate catheter body (10) and configured to control expansion and contraction of the spiral tip or umbrella tip (25).

2. The device of claim 1, wherein the spiral tip comprises at least one complete spiral.

3. The device of claim 1, wherein the spiral tip comprises a plurality of complete spirals.

4. The device of claim 3, wherein gaps between the spirals decrease when the spiral tip is contracted, and wherein gaps between the spirals increase when the spiral tip is expanded.

5. The device of claim 1, further comprising a conducting wire (13) extending from the handle (11) to the spiral tip, wherein the conducting wire is wrapped with a steering spring (15).

6. The device of claim 5, wherein the steering spring (15) is adapted to be controlled through actuation of the handle (11) to expand and contract the spiral tip.

7. The device of claim 6, further comprising a lever (22) connected to the handle (11), wherein extension of the lever (22) causes the steering spring (15) to contract, and wherein constriction of the lever (22) causes the steering spring (15) to expand.

8. The device of claim 1, wherein the spiral tip or umbrella tip (25) is adapted to be expanded and contracted through computer assisted manipulation.

9. The device of claim 1, wherein the spiral tip or umbrella tip (25) is adapted to be expanded and contracted through the use of magnetic fields.

10. The device of claim 1, wherein the handle (11) is further configured to control flexion and extension of the spiral tip or umbrella tip (25).

11. The device of claim 1, wherein the spiral tip is configured to create spiral lesions in tissue and the umbrella tip (25) is configured to create umbrella shaped lesions in tissue.

12. The device of claim 1, wherein the conductive elements comprise conductive plates (21).

13. The device of claim 1, wherein the conductive elements (20, 21) comprise conductive coils (20).

14. The device of claim 1, wherein the spiral tip or umbrella tip (25) is adapted to map the tissue to identify aberrant circuit paths in the tissue.

15. The device of claim 14 wherein the conductive elements (20, 21) comprise a bipolar configuration.

16. The device of claim 14, wherein the conductive elements (20, 21) comprise a unipolar configuration.

17. The device of claim 14, wherein the conductive elements (20, 21) comprise a unipolar and bipolar configuration.

18. The device of claim 1, wherein the spiral tip assumes a substantially planar configuration in an expanded configuration.

19. The device of claim 1, wherein the umbrella tip (25) further comprises a central post (26) extending from the distal end of the catheter body (10), wherein the outer arms (27) extend from a base of the central post (26) to a top of the central post (26).

20. The device of claim 19, further comprising a conducting wire (13), wherein the conducting wire (13) is attached to the handle (11) and interfaces with the central post (26).

21. The device of claim 20, wherein the conducting wire (13) is controlled through manipulation of the handle (11) to expand and contract the umbrella tip (25).

22. The device of claim 21, further comprising a lever (22) connected to the handle (11), wherein extension of the lever (22) causes the central post (26) to extend outside the catheter body (10) and contract the umbrella tip (25), wherein constriction of the lever (22) causes the central post (26) to withdraw inside the catheter body (10) and expand the umbrella tip (25).

23. The device of claim 22, wherein the outer arms (27) bend at hinges as the umbrella tip (25) is expanded.

24. The device of claim 1, wherein the umbrella tip (25) comprises at least three outer arms (27).

## Patentansprüche

1. Vorrichtung für eine Hochdichtekartierung und Gewebeabtragung, umfassend:
- einen langgestreckten Katheterkörper (10) mit einem proximalen Ende und einem distalen Ende;
- einer Spitze in Spiralform oder einer Spitze in Schirmform (25) mit einer Vielzahl von Außenarmen (27), die angeordnet sind um sich radial von einem gemeinsamen Punkt außerhalb des Katheterkörpers zu öffnen, wobei besagte Spitze in Spiralform oder Spitze in Schirmform (25) vom distalen Ende des Katheterkörpers (10) einsetzbar ist;
- an der Spitze in Spiralform oder den Außenarmen (27) der Spitze in Schirmform (25) angeordnete und zur Kartierung und Gewebeabtragung eingerichtete Leitelemente (20, 21);
wobei die Spitze in Spiralform oder Spitze in Schirmform 6 bis 30 Leitelemente (20, 21) umfasst, die über eine Fläche in einem Bereich von 1 cm² bis 12 cm² verteilt sind und wobei besagte Leitelemente (20, 21) eine maximale Länge oder andere Abmessung in einem Bereich von 2 mm bis 20 mm haben und voneinander über eine Distanz in einem Bereich von 1 mm bis 10 mm beabstandet sind; und
- einen am proximalen Ende des langgestreckten Katheterkörpers (10) angeordneten Griff (11), der eingerichtet ist die Expansion und Kontraktion der Spitze in Spiralform oder der Spitze in Schirmform (25) zu kontrollieren.

2. Vorrichtung nach Anspruch 1, wobei die Spitze in Spiralform mindestens eine vollständige Spirale umfasst.

3. Vorrichtung nach Anspruch 1, wobei die Spitze in Spiralform eine Vielzahl an vollständigen Spiralen umfasst.

4. Vorrichtung nach Anspruch 3, wobei sich die Spalten zwischen den Spiralen verkleinern, wenn die Spitze in Spiralform kontrahiert wird und wobei sich die Spalten zwischen den Spiralen vergrößern, wenn die Spitze in Spiralform expandiert wird.

5. Vorrichtung nach Anspruch 1, des Weiteren umfassend einen sich von dem Griff (11) zu der Spitze in Spiralform erstreckenden Leitdraht (13), wobei der Leitdraht mit einer Lenkfeder (15) umhüllt ist.

6. Vorrichtung nach Anspruch 5, wobei die Lenkfeder (15) eingerichtet ist um durch Betätigung des Griffs (11) eine Expansion und Kontraktion der Spitze in Spiralform zu steuern.

7. Vorrichtung nach Anspruch 6, des Weiteren umfassend einen mit dem Griff (11) verbundenen Hebel (22), wobei die Weiterung des Hebels(22) eine Kontraktion der Lenkfeder (15) bewirkt und die Engerstellung des Hebels (22) eine Ausdehnung der Lenkfeder (15) bewirkt.

8. Vorrichtung nach Anspruch 1,wobei die Spitze in Spiralform oder Spitze in Schirmform (25) eingerichtet ist um durch computergestützte Manipulation expandierbar und kontrahierbar zu sein.

9. Vorrichtung nach Anspruch 1, wobei die Spitze in Spiralform oder Spitze in Schirmform (25) eingerichtet ist um durch die Anwendung von Magnetfeldern expandierbar und kontrahierbar zu sein.

10. Vorrichtung nach Anspruch 1, wobei der Griff (11) des Weiteren eingerichtet ist die Beugung und Verlängerung der Spitze in Spiralform oder Spitze in Schirmform (25)zu steuern.

11. Vorrichtung nach Anspruch 1, wobei die Spitze in Spiralform eingerichtet ist spiralförmige Läsionen im Gebe zu erzeugen und die Spitze in Schirmform (25) eingerichtet ist schirmförmige Läsionen im Gewebe zu erzeugen.

12. Vorrichtung nach Anspruch 1, wobei die Leitelemente Leitplatten (21) umfassen.

13. Vorrichtung nach Anspruch 1, wobei die Leitelemente (20, 21) Leitspulen (20) umfassen.

14. Vorrichtung nach Anspruch 1, wobei die Spitze in Spiralform oder die Spitze in Schirmform (25) angepasst ist um das Gewebe zu kartieren um fehlerhafte Leitungswege in dem Gewebe zu identifizieren.

15. Vorrichtung nach Anspruch 14, wobei die Leitelemente (20, 21) eine bipolare Konfiguration aufweisen.

16. Vorrichtung nach Anspruch 14, wobei die Leitelemente (20, 21) eine unipolare Konfiguration aufweisen.

17. Vorrichtung nach Anspruch 14, wobei die Leitelemente (20, 21) eine unipolare und bipolare Konfiguration aufweisen.

18. Vorrichtung nach Anspruch 1, wobei die Spitze in Spiralform in expandierter Form eine im Wesentlichen ebene Konfiguration aufweist.

19. Vorrichtung nach Anspruch 1, wobei die Spitze in Schirmform (25) des Weiteren eine sich vom distalen Ende des Katheterkörpers (10) erstreckende Zentralstrebe (26) aufweist, wobei sich die Außenarme (27) von einer Basis der Zentralstrebe (26) zu einer Spitze der Zentralstrebe erstrecken (26).

20. Vorrichtung nach Anspruch 19, des Weiteren umfassend einen Leitdraht (13), wobei der Leitdraht (13) an dem Griff (11) befestigt ist und mit der Zentralstrebe (26) verbunden ist.

21. Vorrichtung nach Anspruch 20, wobei der Leitdraht (13) durch Betätigung des Griffs (11) kontrolliert wird um die Spitze in Schirmform (25) zu expandieren und zu kontrahieren.

22. Vorrichtung nach Anspruch 21, des Weiteren umfassend einen mit dem Griff (11) verbundenen Hebel (22), wobei die Weitung des Hebels (22) die Erstreckung der Zentralstrebe (26) aus dem Katheterkörper (10) heraus und das Zusammenziehens der Spitze in Schirmform (25) bewirkt, wobei die Engerstellung des Hebels (22) das Zurückziehen der Zentralstrebe (26) in das Innere des Katheterkörpers (10) und die Expansion der Spitze in Schirmform (25) bewirkt.

23. Vorrichtung nach Anspruch 22, wobei die Außenarme (27) sich an Scharnieren abwinkeln, wenn die Spitze in Schirmform (25) expandiert wird.

24. Vorrichtung nach Anspruch 1, wobei die Spitze in Schirmform (25) mindestens drei Außenarme (27) umfasst.

## Revendications

1. Dispositif pour la cartographie haute densité et pour l'ablation de tissus, comprenant :
- un corps de cathéter allongé (10) ayant une extrémité proximale et une extrémité distale ;
- une pointe spiralée ou une pointe de type parapluie (25) ayant une pluralité de bras extérieurs (27) agencés pour s'ouvrir radialement à partir d'un point commun à l'extérieur du corps de cathéter, ladite pointe spiralée ou pointe de type parapluie (25) étant déployable depuis l'extrémité distale du corps de cathéter (10) ;
- des éléments conducteurs (20, 21) disposés sur la pointe spiralée ou sur les bras extérieurs (27) de la pointe de type parapluie (25) et configurés pour cartographier et effectuer l'ablation de tissus ;
dans lequel la pointe spiralée ou la pointe de type parapluie comprend de 6 à 30 éléments conducteurs (20, 21) distribués sur une zone dans la plage de 1 cm² à 12 cm² et dans lequel lesdits éléments conducteurs (20, 21) ont une longueur maximale ou une autre dimension dans la plage de 2 mm à 20 mm et sont espacés d'une distance dans la plage de 1 mm à 10 mm ; et
- une manette (11) disposée à l'extrémité proximale du corps de cathéter allongé (10) et configurée pour commander le déploiement et la contraction de la pointe spiralée ou de la pointe de type parapluie (25).

2. Dispositif selon la revendication 1, dans lequel la pointe spiralée comprend au moins une spirale complète.

3. Dispositif selon la revendication 1, dans lequel la pointe spiralée comprend une pluralité de spirales complètes.

4. Dispositif selon la revendication 3, dans lequel les espaces entre les spirales diminuent lorsque la pointe spiralée est contractée, et dans lequel les espaces entre les spirales augmentent lorsque la pointe spiralée est déployée.

5. Dispositif selon la revendication 1, comprenant en outre un fil conducteur (13) s'étendant de la manette (11) à la pointe spiralée, dans lequel le fil conducteur est enveloppé d'un ressort directeur (15).

6. Dispositif selon la revendication 5, dans lequel le ressort directeur (15) est adapté pour être commandé par l'actionnement de la manette (11) pour déployer et contracter la pointe spiralée.

7. Dispositif selon la revendication 6, comprenant en outre un levier (22) relié à la manette (11), dans lequel l'extension du levier (22) entraîne la contraction du ressort directeur (15), et dans lequel la constriction du levier (22) entraîne le déploiement du ressort directeur (15).

8. Dispositif selon la revendication 1, dans lequel la pointe spiralée ou la pointe de type parapluie (25) est adaptée pour être déployée et contractée par une manipulation assistée par ordinateur.

9. Dispositif selon la revendication 1, dans lequel la pointe spiralée ou la pointe de type parapluie (25) est adaptée pour être déployée et contractée par l'utilisation de champs magnétiques.

10. Dispositif selon la revendication 1, dans lequel la manette (11) est en outre configurée pour commander la flexion et l'extension de la pointe spiralée ou de la pointe de type parapluie (25).

11. Dispositif selon la revendication 1, dans lequel la pointe spiralée est configurée pour créer des lésions en spirale dans un tissu et la pointe de type parapluie (25) est configurée pour créer des lésions en forme de parapluie dans un tissu.

12. Dispositif selon la revendication 1, dans lequel les éléments conducteurs comprennent des plaques conductrices (21).

13. Dispositif selon la revendication 1, dans lequel les éléments conducteurs (20, 21) comprennent des bobines conductrices (20).

14. Dispositif selon la revendication 1, dans lequel la pointe spiralée ou la pointe de type parapluie (25) est adaptée pour cartographier le tissu pour identifier des trajets de circuit aberrants dans le tissu.

15. Dispositif selon la revendication 14, dans lequel les éléments conducteurs (20, 21) comprennent une configuration bipolaire.

16. Dispositif selon la revendication 14, dans lequel les éléments conducteurs (20, 21) comprennent une configuration unipolaire.

17. Dispositif selon la revendication 14, dans lequel les éléments conducteurs (20, 21) comprennent une configuration unipolaire et bipolaire.

18. Dispositif selon la revendication 1, dans lequel la pointe spiralée adopte une configuration sensiblement plane dans une configuration déployée.

19. Dispositif selon la revendication 1, dans lequel la pointe de type parapluie (25) comprend en outre un montant central (26) s'étendant depuis l'extrémité distale du corps de cathéter (10), dans lequel les bras extérieurs (27) s'étendent depuis une base du montant central (26) vers un sommet du montant central (26).

20. Dispositif selon la revendication 19, comprenant en outre un fil conducteur (13), dans lequel le fil conducteur (13) est attaché à la manette (11) et assure l'interface avec le montant central (26).

21. Dispositif selon la revendication 20, dans lequel le fil conducteur (13) est commandé par la manipulation de la manette (11) pour déployer et contracter la pointe de type parapluie (25).

22. Dispositif selon la revendication 21, comprenant en outre un levier (22) relié à la manette (11), dans lequel l'extension du levier (22) entraîne l'extension du montant central (26) à l'extérieur du corps de cathéter (10) et la contraction de la pointe de type parapluie (25), et dans lequel la constriction du levier (22) entraîne le retrait du montant central (26) à l'intérieur du corps du cathéter (10) et le déploiement de la pointe de type parapluie (25).

23. Dispositif selon la revendication 22, dans lequel les bras extérieurs (27) se plient au niveau de charnières lorsque la pointe de type parapluie (25) est déployée.

24. Dispositif selon la revendication 1, dans lequel la pointe de type parapluie (25) comprend au moins trois bras extérieurs (27).
